Europäisches Patentamt

European Patent Office

Office européen des brevets

(19)

(11) EP 1 338 301 A1

(12) EUROPEAN PATENT APPLICATION

(43) Date of publication:
27.08.2003 Bulletin 2003/35

(51) Int Cl.7: A61N 1/36

(21) Application number: 02447028.8

(22) Date of filing: 21.02.2002

(84) Designated Contracting States:
AT BE CH CY DE DK ES FI FR GB GR IE IT LI LU MC NL PT SE TR
Designated Extension States:
AL LT LV MK RO SI

(71) Applicant: Govaerts, Paul J. M.
2360 Antwerpen-Wilrijk (BE)

(72) Inventor: Govaerts, Paul J. M.
2360 Antwerpen-Wilrijk (BE)

(74) Representative:
Brants, Johan Philippe Emile et al
De Clercq, Brants & Partner cv
Edgard Gevaertdreef 10a
9830 Sint-Martens-Latem (BE)

(54) **Method for automatic fitting of cochlear implants, obtained cochlear implant and computer programs therefor**

(57) The present invention relates to a method for calibrating (fitting) a cochlear implant (C1) based on selected data from the medical history of the patient prior to the moment of fitting, and based on audiological feedback tests.

The invention further relates to a computer program, stored on a computer readable medium comprising a computing routine transforming data corresponding a patient's medical history data using an algorithm (s), to a new data set that corresponds to parameters for a C1 adjustment, the results given as readable output and/or a file of any format and/or data passed to other computing routine(s).

EP 1 338 301 A1

**Description**

**Field of the Invention**

The present invention relates to a method for automatically fitting (adjusting) a cochlear implant. It also relates to the cochlear implant fitted according to the method. It further relates to computer programs as used in the method.

**Background of the Invention**

A cochlear implant (Cl) is an electronic medical device, implantable in the cochlea, which directly stimulates the auditory nerve. Profoundly deaf patients with a Cl benefit from a sensation of hearing. Typically a Cl system comprises a microphone (to detect sound), a speech processor (for converting sound signals into electrical signals appropriate to stimulate the auditory nerve), and an implant containing an electrode array (a number of wires and contact surfaces to bring electrical current to the proximity of the auditory nerve). In its actual form the microphone and speech processor are not part of the implant element, but are worn or carried by the patient and communicate with the Cl *via* an inductive, radio frequency or other wireless link.

Cl devices that are known in the art require manual "fitting" *i.e.* the adjustment of parameters in the speech processor component such that sounds perceived by the patient are representational and at a comfortable level. The parameters available for adjustment can vary from device to device but they may include one or more of the following:

- the number of active channels (related to the number of active electrodes in the array)
- the subjective or objective perceptive thresholds per channel (often called the T-level)
- the subjective or objective most comfortable or uncomfortable intensity level (often called the C-level)
- the gain per channel
- the frequency of stimulation
- the type of speech algorithm
- the frequency definition of the different channels
   There are currently three major manufacturers of Cl devices: Cochlear Ltd who manufacture the "Nucleus" Cl, Advanced Bionic Systems (USA) who manufacture the "Clarion" Cl, and Med-EI (Austria) who manufacture the "Med-EI" Cl.

Manual fitting, currently the norm, is technically demanding and time consuming. The patient is required to answer questions in response to a series of audiological tests, the results of which are used to make an adjustment to the Cl. The tests are repeated again and further adjustments made as necessary. Several adjustments need to be made during the course of the first year of operation requiring at least 30 man hours (adult patients) or at least 60 hours (infant patients) of the fitter's time. Thereafter patients need to have the device adjusted on an annual basis, consuming between 10 and 20 man hours per annum. Furthermore, the accuracy of the test varies according to the patient's background - those who have never heard a sound before and children may require even longer fitting times. A cochlear implant device that can automate the fitting procedure without reliance on interactive subjective feedback would save hundreds of man hours in labour, resources and money during the lifetime of the device.

Fitting is an expert skill; the availability of those skilled in the art is limited to mainly developed countries. A way of simplifying the fitting procedure, so that a technician could competently and safely perform the calibration would make Cl implants available to countries without specialist audiological resources.

In the prior art a type of self-fitting Cl device has been disclosed in patent number US 6,157,861 which comprises the following essential features:

- a sensor for detecting the evoked potential due to stimulation of the electrode array.
- a sensor for detecting a middle ear reflex (*stapedius* or *tensor tympani* reflex).
- a microcontroller for controlling the electrode array, the evoked potential sensor and the middle ear reflex detector.

The *evoked potential* and *middle ear reflex,* physiological signals generated by the nervous system or middle ear muscles in response to stimulation of the auditory nerve, are measured in the Cl fitting procedure which comprises a set of steps, thus:

1) the electrode array stimulates the cochlear at a particular intensity value.
2) the effect on the stapedius reflex and the evoked potential are detected.
3) the results are analysed using a 'yes/no' logical flow chart.
4) the intensity of stimulation is adjusted up or down.

5) stimulation is repeated (steps 1-4) until a desired response occurs.

6) the final stimulation level is used to derive the intensity of stimulation during normal use of the IC device.

The result of the procedure is that upper and lower limits can be placed on the intensity of stimulation of each channel (stimulation threshold) and the most comfortable loudness can be determined.

Compared with the standard fitting method, US Pat. No. 6,157,861 requires additional special sensors and circuitry adding to the expense of the device, and the procedure automates fitting for only certain parameters.

Known in the art is a method for making a CI adjustment based on Neural Response Telemetry (NRT), disclosed in PCT Application No. WO 00/52963. Generally, NRT measures electrical potential signals generated after the delivery of an electrical stimulus to a set of two or more electrodes contacting the fibers of the auditory nerve. Information regarding the temporal-, spatial- and spatio-temporal-properties can be determined by NRT. The invention in PCT App. No. WO 00/52963 is based on the discovery that some parameters of the NRT response can be used to predict which patients would benefit from a certain frequency of stimulation. The invention predicts from the NRT response whether a 'high-rate stimulation' or a 'low-rate stimulation' algorithm is suitable. Compared with the standard method of fitting, PCT App. No. WO 00/52963 requires additional circuitry to measure the NRT response, and the technique provides only one CI parameter - the rate of electrode stimulation.

The PCT Application No. WO 97/43871 (EP0906713A1) discloses a method of predicting those CI parameters concerned with the frequency allocation of each electrode; the position of the stimulating electrode in the cochlear correlates with the frequency perceived upon stimulation. For example, it is known that high frequency sounds are perceived by stimulating the basally-located electrodes. The method of PCT App. No. WO 97/43871 requires the use of medical imaging (*e.g.* X-rays) in order to visualize the implant *in situ*, and a manual analysis of the image to obtain the frequency range information.

The US Patent No. 5,626,629 describes a computer-assisted method of fitting which is based on *stapedius* reflex measurements and electrically elicited auditory brainstem responses. The CI speech processor is coupled to a computer running a fitting program which has a number of distinct features including:

1) A patient database.
2) Prompts for measuring *stapedius* reflex.
3) Prompts for measuring electrically elicited auditory brainstem response (EABR).
4) Calculation of T-level value based on 2) and 3).
5) Calculation C-level value based on 2) and 3).
6) Prompts for manual fine-tuning.
7) Test sweep of implant.
8) Test of implant balance.
9) Real time speech synthesizer with sound-processing features.
10) Display of audiological impedances.
11) Adjustment of pulse width, firing sequence and repetition rate.

The computer program described in US Pat. No. 5,626,629 facilitates manual fitting by providing a *real-time* interface with the CI in respect of the features above. Fitting using US Pat. No. 5,626,629 still requires extensive interactions by the skilled fitter.

Known the art is the EP Patent No. 0885548 (equivalent to US 6,205,360) which features sensors to detect evoked neural potentials and *stapedius* muscle activity in response stimulation by an electrode array. As described for US Pat. No. 6,157,861, this arrangement of sensors may be used to set the T- and C-levels. In a preferred embodiment of EP 0885548, the sensors are worn in normal use and are coupled to the speech processor so that C- and T-levels can be automatically set by the patient at any time by the touch of a button. The invention requires additional speech processor circuitry and a *stapedius* muscle sensor implant.

**Objectives of the invention**

There is a need for an automatic fitting procedure that can be applied to standard cochlear implant devices such as the Nucleus, Clarion, Med-EI and others, and which overcomes the disadvantages of the prior art. The present invention is based on the discovery that patient medical history data correlates with parameters required for a CI adjustment. Thus, the present invention is a method for fitting a CI device using data from the patient's medical history to produce a set of optimised calibration values for transfer to the CI. The CI, operating with values from the invention, needs only minor adjustment thereafter using a set of audiological tests. There is no requirement for the patient to undergo the tedious process of manual adjustment or to have additional surgical intrusion.

## Summary of the invention

The invention comprises a method for calibrating (fitting) a cochlear implant based on data from the medical history of the patient prior to the moment of fitting and based audiological feedback tests, removing the need for time-consuming manual fitting or additional specialised electronics.

## Definitions

*Fitting* - adjustment or calibration of the CI to match the patient's anatomical, psychophysical and perceptive characteristics. The terms fitting, adjustment and calibration are interchangeable.
*Manual fitting* - fitting, as above, using the patient's subjective answers to audiological tests as a basis for calibration values.
*Manual input* - physical means of entering data into a computer - for example by keyboard, any interactive input means, dictation.

## Detailed description of the invention

The invention comprises an 'historical-data method' for transforming data from the patient's medical history prior to the moment of fitting, using an algorithm or collection of algorithms, into a data set corresponding to CI calibration parameters. Thus, the fitting time is considerably reduced since the historical-data calibration method requires no tedious real-time measurements.
The data from the medical history comprises one or more items from a list including:

- approximate date of onset of deafness.
- type of deafness from a list comprising congenital-, prelingual acquired-, postlingual progressive-, postlingual sudden-deafness.
- date on which hearing loss was first documented by medical/paramedical staff.
- date on which the ear was last stimulated.
- Etiology (cause) most likely for hearing loss .
- Results of audiometric tests prior to the implantation.
- Maximal word discrimination in speech audiometry test.
- Speech audiometry in noise (0 dB S/N): maximal word discrimination score.
- Results of intraoperative objective measurements, such as NRT

In one embodiment, the historical-data calibration method comprises the following steps:

a) Transformation of selective data from the patient's medical history into a data set (*data 1*) using at some stage an algorithm(s) and
b) Reformatting of *data 1* and transfer of reformatted data to the CI for subsequent use.

In one embodiment, the algorithm(s) used by the historical-data calibration comprises a set of formulae which transforms patient medical history data into values corresponding to a **CI adjustment for the T-level parameter.** Of the said set of formulae, the formula [1] below is one such variation used to calibrate the T-level parameter in a patient with a CI in the right ear, from the medical history data shown in Table 1.

$$T01EE = A_{EE} + I041B + I042C + I043D$$
$$- Int\left[10\times\left(\frac{E + I041F + I042G + I043H - I044J}{15} + 1\right)\right] \quad [1]$$

*Formula used to calculate T01EE values for a patient with a cochlear implant of N electrodes (EE = 01 to N). The values of I041, I042, I043 and I044 are taken from the patient history in Table 1. Int is an abbreviation for 'integer' meaning round down to the lower integer.*
The CI digital sound processor requires parameter values in the form of T*nnEE*, where *nn* is the series number, and *EE* is the electrode number. Formula [1] provides values of T01EE (*i.e.* when *nn* = 01).

Constants A to J in formula [1] are under constant improvement as the quality and quantity of patient statistical information develops over time. In one embodiment, A is a value in the range 1 to 100, offset by k*EE* (where k is an integer in the range -10 to 10); B is a decimal fraction in the range 0.001 to 0.999; C is a decimal fraction in the range 0.001 to 0.999; D is a decimal fraction in the range 0.001 to 0.999; E is a decimal fraction in the range -0.999 to 0.999; F is a decimal fraction in the range 0.001 to 0.999; G is a decimal fraction in the range 0.001 to 0.999; H is a decimal fraction in the range 0.001 to 0.999; J is a decimal fraction in the range 0.001 to 0.999

In another embodiment of formula [1], A is a value in the range 50 to 100, offset by k*EE* (where k is an integer in the range -5 to 5); B is a decimal fraction in the range 0.001 to 0.100; C is a decimal fraction in the range 0.100 to 0.500; D is a decimal fraction in the range 0.100 to 0.500; E is a decimal fraction in the range -0.999 to 0.100; F is a decimal fraction in the range 0.001 to 0.100 G is a decimal fraction in the range 0.001 to 0.500; H is a decimal fraction in the range 0.001 to 0.500; J is a decimal fraction in the range 0.001 to 0.500.

Another formula, in the set of formulae for calculating the T-level, is a variation which provides the values of TnnEE, for the series T*02*EE to T*nn*EE where nn>01 (formula [2]).

$$TnnEE = T(nn-1)EE + \sum_{n=40}^{n=01} Fn\{n\}$$  [2]

*Formula used to calculate the TnnEE values for a series of nn. Fn{n} are functions which operate on the respective elements of the patient data given in Table 1 (see below).*

Functions Fn (where n is 1, 2, 3.....40) in formula [2] operate on patient data I(n) in Table 1, according to an embodiment of a historical-data calibration algorithm, formula [3].

$$Fn\{n\} = [An + Bn \times [I(n)]^{Cn}]^{Dn}$$  [3]

Where *n* is the item number of patient medical data taken from Table 1; Formula [3] is under constant improvement as the quality and quantity of patient statistical information develops over time. In one embodiment. *An, Bn, Cn* and *Dn* are constants. *An* is a value in the range - 100 to 100; *Bn* is a value in the range -100 to 100, *Cn* is a value in the range -100 to 50, *Dn* is a value in the range -100 to 50.

The matrix of values TnnEE, the result of the both transformations above, represents a set of operational CI **T-level** values which, after reformatting, can be loaded directly into the digital speech processor of the CI.

The invention also comprises a method ('feedback-calibration') for calibrating (fitting) a cochlear implant (CI) based on audiological tests given with the CI *in situ* and operating with any parameter set.

In one embodiment, the feedback-calibration method comprises transforming data from both i) audiological tests given with the CI *in situ*, operating with any parameter set, and ii) the said operating parameter set, using an algorithm or collection of algorithms, into a new data set corresponding to CI calibration parameters.

The said audiological tests comprise one or more from a list including: audiometrical thresholds, scores on word- or sentence tests, subjective sound appreciation by the subject, scores on APE® (Auditory Phoneme Evaluation, ©Melakos nv, Antwerp, Belgium).

In another embodiment, the feedback-calibration method comprises the following steps:

a) audiological tests with the CI *in situ* and operating with any parameter set (*e.g. data 1*) and
b) transformation of data from both i) the results of the said audiological tests and ii) the CI operating parameter set into a new data set *(data 2),* using at some stage an algorithm(s) and
c) reformatting of *data 2* and transfer of reformatted data to the CI for use, and/or
d) optionally repeating the audiological tests and subsequent steps (a-d) until no improvement.

In one embodiment, the algorithm(s) used by the feedback-calibration method comprises a set of formulae which transforms data from both i) the results of audiological tests performed with the CI *in situ* and ii) the CI operating parameters into values corresponding to a CI adjustment for the **T-level** parameter. Of the said set of formulae, the formula [4] below is one such variation used to calibrate the T-level parameter in a patient with a CI in the right ear, using results from the APE phoneme discrimination tests. As for the above embodiment, the CI requires T-level values in the form of TnnEE.

$$TmmNN = TaaNN + A \qquad\qquad [4]$$

*Formula used to calculate a modified T-level parameter set (TmmNN) using values from the results of audiological tests (A) and the operating parameter set (TaaNN)*

In another embodiment, the historical-data calibration method comprises the transformation of data by an algorithm(s), performed using a computer program, stored on a computer readable medium.

In another embodiment, the feedback-calibration calibration method comprises the transformation of data by an algorithm(s), performed using a computer program, stored on a computer readable medium.

The invention comprises an 'export-method', using computing routines stored on a computer readable medium, for formatting the data sets - held as a file of any format, and/or using data from another computing routine(s) corresponding to any parameters for a CI adjustment - to data sets suitable for direct transfer to the digital memory of the CI speech processor, and/or to a file for reading by the CI's manufacturer's software, and/or to a file of any format, and/or to another computing routine(s), so saving the time-consuming task of manually inputting parameters into the CI.

In another embodiment, the historical-data calibration method comprises the use of a computer program comprising a computing routine(s), stored on a computer readable medium for providing a means for entry of patient history data in any format by manual input, and/or by reading a file held on a computer readable medium and/or by accepting data from another computing routine(s) for formatting and transfer to a file of any format, and/or to another computing routine(s) for the purpose of transformation by the historical-data calibration algorithm(s), so providing a easy-to-use, common interface between all patient data types and the said algorithm.

In another embodiment, the feedback-calibration method comprises the use a computer program comprising a computing routine(s), stored on a computer readable medium for providing a means for entry of results of audiological tests made with the CI *in situ,* by manual input and/or by reading a file held on a computer readable medium for formatting and transfer to a file of any format, and/or to another computing routine(s) for the purpose of transformation by the feedback-calibration algorithm(s), so providing an easy-to-use, common interface between all types of test results data and the said algorithm(s).

In another embodiment, the feedback-calibration method comprises the use of a computer program comprising a computing routine(s), stored on a computer readable medium for providing a means for entry of operational CI parameters by manual input and/or entry by reading a file held on a computer readable medium and/or by accepting data from another computing routine(s) for formatting and transfer to a file of any format, and/or to another computing routine(s) for the purpose of transformation by the feedback calibration algorithm.

In another embodiment, the historical-data calibration method comprises the use of a computer program comprising a computing routine(s), stored on a computer readable medium for transforming a patient's medical history data held as a file in any format and/or as data passed from another computing routine(s), using an algorithm(s), to a new data set that corresponds to parameters for a CI adjustment, the results given as readable output and/or a file of any format and/or data passed to other computing routine(s).

In another embodiment, the feedback-calibration method comprises the use of a computer program comprising a computing routine(s), stored on a computer readable medium for transforming data held as a file in any format and/or as data passed from another computing routine(s), that corresponds to i) the results of audiological tests made with the CI *in situ* and operating with any CI parameter set and ii) data from the said CI operational parameter set, to a new data set that corresponds to parameters for a new CI adjustment using the said algorithm(s), the results given as readable output and/or a file of any format and/or data passed to other computing routine(s).

A computer program, stored on a computer readable medium for transforming a patient's medical history data using an algorithm(s), to a new data set that corresponds to parameters for a CI adjustment, the results given as readable output and/or a file of any format and/or data passed to other computing routine(s), facilitating the historical-data calibration method.

A computer program, stored on a computer readable medium for transforming data from both i) the results of audiological tests made with the CI *in situ* and operating with any CI parameter set, and ii) data from the said CI operational parameter set, using an algorithm(s), to a new data set that corresponds to parameters for a new CI adjustment, the results given as readable output and/or a file of any format and/or data passed to other computing routine(s), facilitating the feedback-calibration method.

A computer program, stored on a computer readable medium, for reformatting data sets corresponding to parameters for a CI adjustment, to data sets suitable for direct transfer to the digital memory of the speech processor, and/or to a file for reading by the CI's manufacturer's software, and/or to a file of any format, and/or to another computing routine(s), facilitating the export-method.

In another embodiment, a CI device calibrated according to any of the methods above reduces the fitting time required compared with manual calibration.

**Figures**

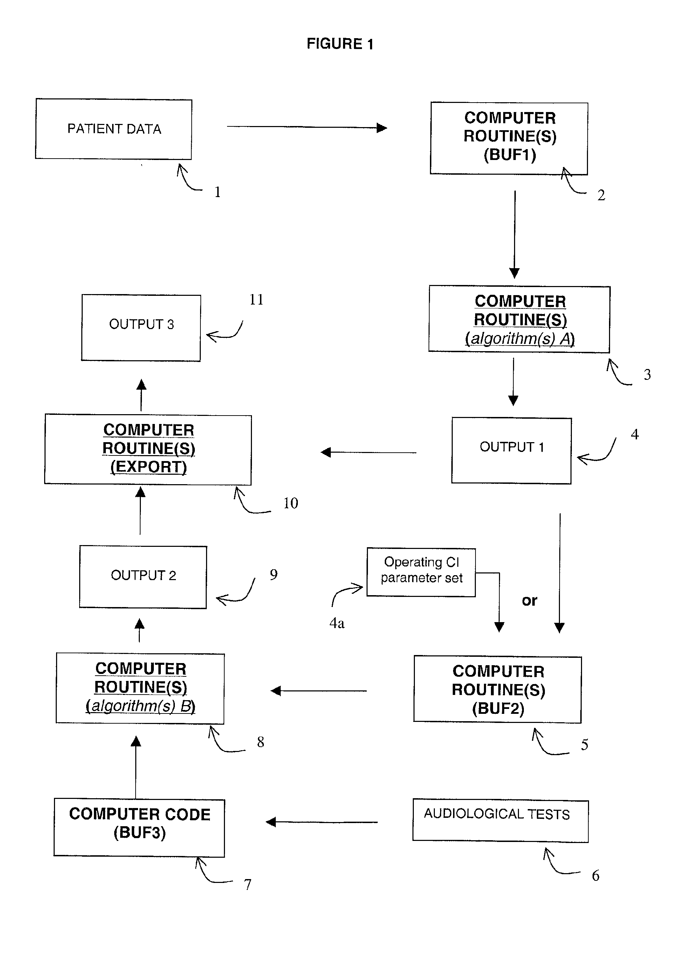

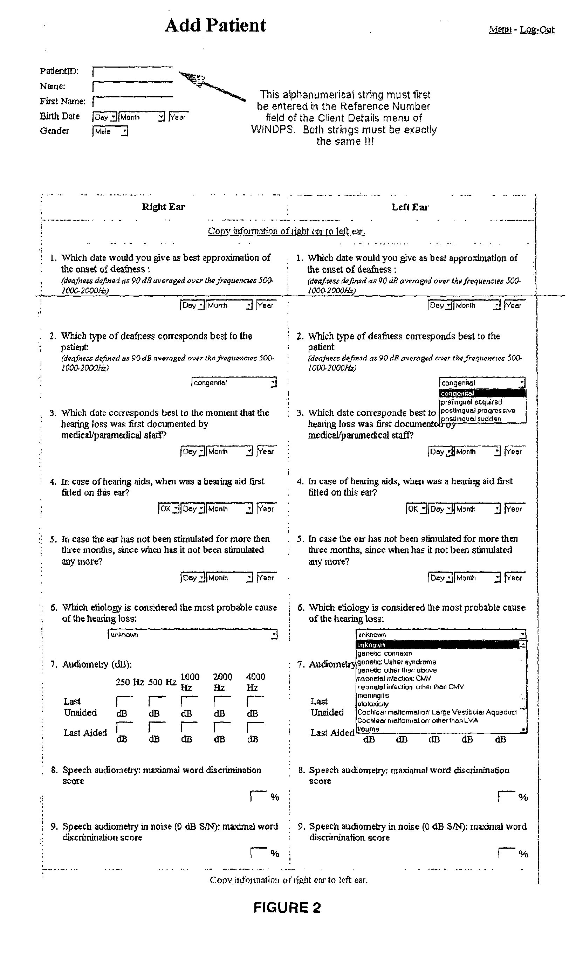

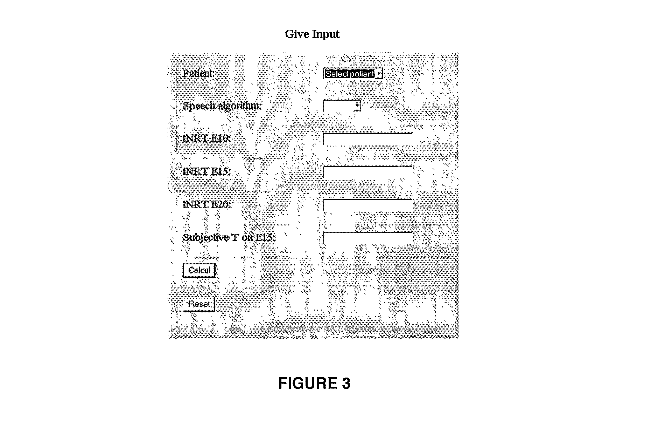

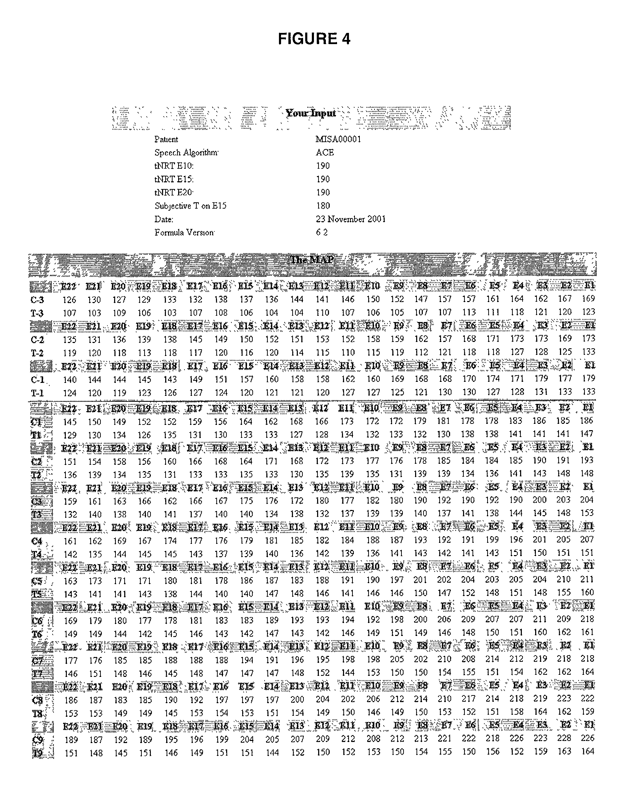

Computing routine(s) (BUF1, Box 2, Figure 1) accepts data from the patient's medical history in the form of manual entry (*e.g.* keyboard, interactive input means, dictation; Figures 2 and 3 as examples), and/or text file held on a computer readable medium, and/or a binary file held on a computer readable medium, and/or any readable file held on a computer readable medium (Box 1, Figure 1). The routine formats the data and makes it available to the computing routine(s) that performs *algorithm(s) A* (Box 3, Figure 1). The computing routine(s) that performs *algorithm(s) A* (Box 3, Figure 1) transforms the said formatted patient medical data into a new data set (OUTPUT 1, Box 4, Figure 1, Figure 4 as an example) corresponding to parameters for a CI adjustment.

The computing routine(s) (EXPORT, Box 10, Figure 1) accepts data from OUTPUT 1 (Figure 1) or accepts data of any format corresponding to parameters for a CI adjustment, and reformats it to a new data set (OUTPUT 3, Box 11, Figure 1) for direct transfer to the digital memory of CI speech processor, and/or to a file for reading by the CI's manufacturer's software, and/or to a file of any format, and/or to another computing routine.

AUDIOLOGICAL TESTS (Box 6, Figure 1) comprises data from audiological tests performed with the CI *in situ* and operating with parameters determined by patient medical history or with other parameters. Said data is in the form of manual entry, and/or text file(s) held on a computer readable medium, and/or binary file(s) held on a computer readable medium, and/or any readable file(s) held on a computer readable medium. The computing routine(s) BUF3 (Box 7, Figure 1) accepts the data of Box 6 (Figure 1) and reformats and makes it available to computing routine(s) that performs *algorithm(s) B* (Box 8, Figure 1).

The computing routine(s) that performs *algorithm(s) B* (Box 8, Figure 1) accepts the said formatted audiological data and also accepts the set of operational parameters used in said audiological tests (Box 4, Figure 1 if CI calibrated with patient history data, or Box 4a, Figure 1). The said operational parameters are reformatted by computing routine(s) BUF2 (Box 5, Figure 1) for use by *algorithm(s)* B . The result of *algorithm(s) B* (OUTPUT 2, Box 9, Figure 1; Figure 3 as an example) corresponds to parameters for an improved CI adjustment.

The computing routine(s) (EXPORT, Box 10, Figure 1), the same routine as described above, accepts data from OUTPUT 2 (Box 9, Figure 1) or accepts data of any format corresponding to parameters for a CI adjustment, and reformats it to a new data set (OUTPUT 3, Box 11, Figure 1) for direct transfer to the digital memory of CI speech processor, and/or to a file for reading by the CI's manufacturer's software, and/or to a file of any format, another computing routine(s).

**Example 1**

A 22-electrode right ear 'Nucleus' cochlear implant was fitted on a patient using the historical data calibration method, and a variation of the algorithm(s) used to calibrate the **T-level**. Patient information from Table 1 (1041, 1042, 1043, 1044) was collected from medical records and used in the formula:

$$T01EE = A_{EE} + I041B + I042C + I043D - Int\left[10\times\left(\frac{E + I041F + I042G + I043H - I044J}{15} + 1\right)\right]$$

Where A took the values [62.9 - (2 x *EE*)]; B was 0.047; C was 0.297; D was 0.336; E was - 0.5; F was 0.074; G was 0.297; H was 0.336; J was 1.0; EE was the series of values 01, 02, 03.....22; 1041 was 190; 1042 was 190; 1043 was 190; 1044 was 180.

The series T01EE for values of EE from 01 to 22 (above) were used as arguments for a second algorithm:

$$TnnEE = T(nn-1)EE + \sum_{40}^{01} Fn\{n\}$$

Functions Fn (where n is 1, 2, 3..... 40) were defined the general equation below:

$$Fn\{n\} = [An + Bn \times [I(n)]^{Cn}]^{Dn}$$

Where I(*n*) was the item of patient medical data taken from Table 1; *An, Bn, Cn* and *Dn* were constants specific to the patient medical data item. *An* was a value in the range -100 to 100; *Bn* was a value in the range -100 to 100, *Cn* was a value in the range -100 to 50, *Dn* was a value in the range -100 to 50.

The resulting map for the T-level calibration is shown in table 2.

**Example 2**

Audiological tests were performed on the patient in Example 1 with the 'Nucleus' CI *in situ.* The patient was tested using the APE® phoneme discrimination tests. The operating T-level parameters (*TaaNN*) were modified to *TmmNN* according to the results of the tests. The patient showed good discrimination, except for the phoneme pair /z/-/s/. The operating CI parameters were adjusted thus:

$$Tmm01=Taa01$$

$$Tmm02=Taa02$$

$$Tmm03=Taa03$$

$$Tmm04=Taa04$$

$$Tmm05=Taa05$$

$$Tmm06=Taa06$$

$$Tmm07=Taa07$$

$$Tmm08=Taa08$$

$$Tmm09=Taa09$$

$$Tmm10=Taa10$$

$$Tmm11=Taa11$$

$$Tmm12=Taa12$$

$$Tmm13=Taa13$$

$$Tmm14=Taa14$$

$$Tmm15=Taa15$$

$$Tmm16=Taa16$$

$$Tmm17=Taa17$$

$$Tmm18=Taa18$$

$$Tmm19=Taa19 + 4$$

$$Tmm20=Taa20 + 8$$

$$Tmm21=Taa21 + 12$$

$$Tmm22=Taa22 + 16$$

**Example 3**

Table 3 provides the surprising results of the using invention on patients at St. Augustinus Hospital, University of Antwerp, in terms of time saved compared with manual fitting.

To take one example, manual fitting during the first year took 56 man-hours per patient for children younger than 6 years old. Using the invention (automatic fitting), it took just 5 man-hours. Subsequent annual fitting on the same age group required 22 man-hours per annum; using the invention, it took just 1 man-hour per annum.

Across all age groups, it took 90% less time to fit the CI using the invention compared with manual fitting.

TABLE 1

| PARAMETER NAME | EAR | PATIENT MEDICAL HISTORY DATA |
|---|---|---|
| | | |
| I001 | R | Date of Birth |
| I002 | R | Gender |
| I003 | R | Date of onset of deafness |
| I004 | R | Date of diagnosis of deafness |
| I005 | R | Age of first fitting of hearing aids |
| I006 | R | Duration of deprivation of auditory input (immediately preceding the implantation) |
| I007 | R | Type of deafness (1 = congenital, 2 = prelingual acquired, 3 = postlingual progressive, 4 = postlingual sudden) |
| I008 | R | Etiology of deafness (1 = unknown, 2 = connexin-mutation, 3 = Usher, 4 = other genetic, 5 = neonatal CMV infection, 6 = meningitis, 7 = ototoxic drugs, 8 = large vestibular aqueduct, 9 = other cochlear malformation, 10 = trauma) |
| I009 | R | last unaided audiometrical threshold at 250 Hz |
| I010 | R | last unaided audiometrical threshold at 500 Hz |
| I011 | R | last unaided audiometrical threshold at 1000 Hz |
| I012 | R | last unaided audiometrical threshold at 2000 Hz |
| I013 | R | last unaided audiometrical threshold at 4000 Hz |
| I014 | R | last aided audiometrical threshold at 250 Hz |
| I015 | R | last aided audiometrical threshold at 500 Hz |

TABLE 1   (continued)

| PARAMETER NAME | EAR | PATIENT MEDICAL HISTORY DATA |
|---|---|---|
| I016 | R | last aided audiometrical threshold at 1000 Hz |
| I017 | R | last aided audiometrical threshold at 2000 Hz |
| I018 | R | last aided audiometrical threshold at 4000 Hz |
| I019 | R | maximal word discrimination score on speech audiometry |
| I020 | R | maximal word discriminations core on speech audiometry in noise (0 dB signal to noise ratio). |
| I021 | L | Date of Birth |
| I022 | L | Gender |
| I023 | L | Date of onset of deafness |
| I024 | L | Date of diagnosis of deafness |
| I025 | L | Age of first fitting of hearing aids |
| I026 | L | Duration of deprivation of auditory input (immediately preceding the implantation) |
| I027 | L | Type of deafness (1 = congenital, 2 = prelingual acquired, 3 = postlingual progressive, 4 = postlingual sudden) |
| I028 | L | Etiology of deafness (1 = unknown, 2 = connexin-mutation, 3 = Usher, 4 = other genetic, 5 = neonatal CMV infection, 6 = meningitis, 7 = ototoxic drugs, 8 = large vestibular aqueduct, 9 = other cochlear malformation, 10 = trauma) |
| I029 | L | last unaided audiometrical threshold at 250 Hz |
| I030 | L | last unaided audiometrical threshold at 500 Hz |
| I031 | L | last unaided audiometrical threshold at 1000 Hz |
| I032 | L | last unaided audiometrical threshold at 2000 Hz |
| I033 | L | last unaided audiometrical threshold at 4000 Hz |
| I034 | L | last aided audiometrical threshold at 250 Hz |
| I035 | L | last aided audiometrical threshold at 500 Hz |
| I036 | L | last aided audiometrical threshold at 1000 Hz |
| I037 | L | last aided audiometrical threshold at 2000 Hz |
| I038 | L | last aided audiometrical threshold at 4000 Hz |
| I039 | L | maximal word discrimination score on speech audiometry |
| I040 | L | maximal word discriminations core on speech audiometry in noise (0 dB signal to noise ratio). |
| I041 | R | threshold of the intraoperative NRT-measurement at electrode 10 |
| I042 | R | threshold of the intraoperative NRT-measurement at electrode 15 |
| I043 | R | threshold of the intraoperative NRT-measurement at electrode 20 |
| I044 | R | subjective threshold with the CI on electrode 15 |
| I045 | R | patient weight |
| I046 | R | patient height |
| I047 | R | Length of Cochlea |
|  |  |  |
| I051 | L | threshold of the intraoperative NRT-measurement at electrode 10 |

TABLE 1   (continued)

| PARAMETER NAME | EAR | PATIENT MEDICAL HISTORY DATA |
|---|---|---|
| I052 | L | threshold of the intraoperative NRT-measurement at electrode 15 |
| I053 | L | threshold of the intraoperative NRT-measurement at electrode 20 |
| I054 | L | subjective threshold with the CI on electrode 15 |
| I055 | L | Patient weight |
| I056 | L | Patient height |
| I057 | L | Length of Cochlear |
|  |  |  |

TABLE 2

| | E1 | E2 | E3 | E4 | E5 | E6 | E7 | E8 | E9 | E10 | E11 | E12 | E13 | E14 | E15 | E16 | E17 | E18 | E19 | E20 | E21 | E22 |
|---|----|----|----|----|----|----|----|----|----|-----|-----|-----|-----|-----|-----|-----|-----|-----|-----|-----|-----|-----|
| T01 | 147 | 141 | 141 | 141 | 138 | 138 | 130 | 132 | 133 | 132 | 134 | 128 | 127 | 133 | 133 | 130 | 131 | 135 | 126 | 134 | 130 | 129 |
| T02 | 148 | 148 | 143 | 141 | 136 | 134 | 139 | 139 | 131 | 135 | 139 | 135 | 130 | 133 | 135 | 133 | 133 | 131 | 135 | 134 | 139 | 136 |
| T03 | 153 | 148 | 145 | 144 | 138 | 141 | 137 | 140 | 139 | 139 | 137 | 132 | 138 | 134 | 140 | 140 | 137 | 141 | 140 | 138 | 140 | 132 |
| T04 | 151 | 151 | 150 | 151 | 143 | 141 | 142 | 143 | 141 | 136 | 139 | 142 | 136 | 140 | 139 | 137 | 143 | 145 | 145 | 144 | 135 | 142 |
| T05 | 160 | 155 | 148 | 151 | 148 | 152 | 147 | 150 | 146 | 146 | 141 | 146 | 148 | 147 | 140 | 140 | 144 | 138 | 143 | 141 | 141 | 143 |
| T06 | 161 | 162 | 160 | 151 | 150 | 148 | 146 | 149 | 151 | 149 | 146 | 142 | 143 | 147 | 142 | 143 | 146 | 145 | 142 | 144 | 149 | 149 |
| T07 | 164 | 162 | 162 | 154 | 151 | 155 | 154 | 150 | 150 | 153 | 144 | 152 | 148 | 147 | 147 | 147 | 148 | 145 | 146 | 148 | 151 | 146 |
| T08 | 159 | 162 | 164 | 158 | 156 | 152 | 153 | 150 | 149 | 146 | 150 | 149 | 154 | 151 | 153 | 154 | 153 | 145 | 149 | 149 | 153 | 153 |
| T09 | 164 | 163 | 159 | 152 | 151 | 150 | 155 | 154 | 150 | 153 | 152 | 150 | 152 | 144 | 151 | 151 | 149 | 146 | 151 | 145 | 148 | 151 |

TABLE 3

| Key: Prep - preparation time; FIT - fitting time; EV - evaluation time; ann - annual. | | | | | | | | | |
|---|---|---|---|---|---|---|---|---|---|
| | Time in man hours | | | | | % time saved | | | |
| | Pre p. | FIT 1y | EV 1y | FIT ann. | EV ann. | FIT | EV | 15y | 1y |
| | | | | | | | | | |
| | 0-6 year old age group | | | | | | | | |
| Manual fitting | 13 | 56 | 25 | 22 | 20 | | | | |
| Automatic fitting | 10 | 5 | 4 | 1 | 2 | 97 | 92 | 93 | 80 |
| | | | | | | | | | |
| | 7 - 17 year old age group | | | | | | | | |
| Manual fitting | 13 | 31 | 19 | 17 | 17 | | | | |
| Automatic fitting | 10 | 4 | 4 | 1 | 2 | 96 | 91 | 92 | 72 |
| | | | | | | | | | |
| | 18+ age group | | | | | | | | |
| Manual fitting | 11 | 29 | 16 | 9 | 10 | | | | |
| Automatic fitting | 8 | 4 | 4 | 1 | 2 | 93 | 84 | 87 | 72 |
| | | | | | | | | | |

## Claims

1. A method for calibrating (fitting) a cochlear implant (CI) based on selected data from the medical history of the patient prior to the moment of fitting, and based on audiological feedback tests.

2. A method according to claim 1 for transforming said medical-history data using an algorithm or collection of algorithms, into a data set corresponding to a set of CI calibration parameters.

3. A method according to claim 2 comprising the following steps:

   a) Transformation of said data using said algorithm(s),
   b) Reformatting said transformed data,
   c) Transfer of said reformatted data to CI for subsequent use.

4. A method according to claim 1 for calibrating (fitting) a cochlear implant (CI) based on said audiological tests given with the CI *in situ* and operating with any parameter set.

5. A method according to claim 4 for transforming data from both i) the results of said audiological tests and ii) the operating data set, using an algorithm or collection of algorithms, into a new data set corresponding to CI calibration parameters.

6. A method according to claim 5 comprising the following steps:

   a) Said audiological tests and said data transformations using said algorithm(s) and
   b) Reformatting said transformed data
   c) transfer of said reformatted data to CI for subsequent use, and/or

d) optionally repeating the audiological tests and subsequent steps (a-d) until no improvement.

**7.** A method according to claim 2 comprising the said transformation of said data by the said algorithm(s) performed using a computer program, stored on a computer readable medium.

**8.** A method according to claim 5 comprising the said transformation of said data by the said algorithm(s) performed using a computing program, stored on a computer readable medium.

**9.** A method comprising the use of a computing routine(s), stored on a computer readable medium for formatting the data sets - held as a file of any format, and/or using data from another computing routine(s) corresponding to any parameters for a CI adjustment - to data sets suitable for direct transfer to the digital memory of the CI speech processor, and/or to a file for reading by the CI's manufacturer's software, and/or to a file of any format, and/or to another computing routine(s).

**10.** A method according to claim 7, where said computer program comprises a computing routine(s), stored on a computer readable medium for providing a means for entry of patient history data in any format by manual input, and/or by reading a file held on a computer readable medium and/or by accepting data from another computing routine for formatting and transfer to a file of any format, and/or to another computing routine(s) for the purpose of transformation by said algorithm(s).

**11.** A method according to claim 8 where said computer program comprises a computing routine(s), stored on a computer readable medium for providing a means for entry of results of said audiological tests made with the CI *in situ,* by manual input and/or by reading a file held on a computer readable medium for formatting and transfer to a file of any format, and/or to another computing routine(s) for the purpose of transformation by said algorithm(s).

**12.** A method according to claim 8 where said computer program comprises a computing routine(s), stored on a computer readable medium for providing a means for entry of said operating parameter set by manual input and/or entry by reading a file held on a computer readable medium and/or by accepting data from another computing routine for formatting and transfer to a file of any format, and/or to another piece of computing routine(s) for the purpose of transformation by said algorithm(s).

**13.** A method according to claim 7 where said computer program comprises a computing routine(s), stored on a computer readable medium for transforming said data held as a file in any format and/or using data passed from another computing routine(s), using said algorithm(s), to a new data set that corresponds to parameters for a CI adjustment, the results given as readable output and/or a file of any format and/or data passed to other computing routine(s).

**14.** A method according to claim 8 where said computer program comprises a computing routine(s), stored on a computer readable medium for transforming said data held as a file in any format and/or using data passed from another computing routine(s), to a new data set that corresponds to parameters for a new CI adjustment using the said algorithm(s), the results given as readable output and/or a file of any format and/or data passed to other computing routine(s).

**15.** Computer program, stored on a computer readable medium comprising a computing routine transforming data corresponding a patient's medical history data using an algorithm(s), to a new data set that corresponds to parameters for a CI adjustment, the results given as readable output and/or a file of any format and/or data passed to other computing routine(s).

**16.** Computer program, stored on a computer readable medium comprising a computing routine for transforming data from both i) the results of audiological tests made with the CI *in situ* and operating with any CI parameter set, and ii) data from the said CI operational parameter set, using an algorithm, to a new data set that corresponds to parameters for a new CI adjustment, the results given as readable output and/or a file of any format and/or data passed to another computing routine(s).

**17.** Computer program stored on a computer readable medium, comprising a computing routine for reformatting data sets corresponding to parameters for a CI adjustment, to data sets suitable for direct transfer to the digital memory of the speech processor, and/or to a file for reading by the CI's manufacturer's software, and/or to a file of any format, and/or to another computing routine(s).

**18.** A CI device calibrated according to any of the methods in claims 1-14.

**FIGURE 1**

# Add Patient

PatientID:
Name:
First Name:
Birth Date [Day ▾] [Month ▾] [Year]
Gender [Male ▾]

This alphanumerical string must first be entered in the Reference Number field of the Client Details menu of WINDPS. Both strings must be exactly the same !!!

| Right Ear | Left Ear |
|---|---|

Copy information of right ear to left ear.

| Right Ear | Left Ear |
|---|---|
| 1. Which date would you give as best approximation of the onset of deafness : *(deafness defined as 90 dB averaged over the frequencies 500-1000-2000Hz)* | 1. Which date would you give as best approximation of the onset of deafness : *(deafness defined as 90 dB averaged over the frequencies 500-1000-2000Hz)* |
| [Day ▾][Month ▾] [Year] | [Day ▾][Month ▾] [Year] |
| 2. Which type of deafness corresponds best to the patient: *(deafness defined as 90 dB averaged over the frequencies 500-1000-2000Hz)* | 2. Which type of deafness corresponds best to the patient: *(deafness defined as 90 dB averaged over the frequencies 500-1000-2000Hz)* |
| [congenital ▾] | [congenital ▾] congenital / prelingual acquired / postlingual progressive / postlingual sudden |
| 3. Which date corresponds best to the moment that the hearing loss was first documented by medical/paramedical staff? | 3. Which date corresponds best to the moment that the hearing loss was first documented by medical/paramedical staff? |
| [Day ▾][Month ▾] [Year] | [Day ▾][Month ▾] [Year] |
| 4. In case of hearing aids, when was a hearing aid first fitted on this ear? | 4. In case of hearing aids, when was a hearing aid first fitted on this ear? |
| [OK ▾][Day ▾][Month ▾] [Year] | [OK ▾][Day ▾][Month ▾] [Year] |
| 5. In case the ear has not been stimulated for more then three months, since when has it not been stimulated any more? | 5. In case the ear has not been stimulated for more then three months, since when has it not been stimulated any more? |
| [Day ▾][Month ▾] [Year] | [Day ▾][Month ▾] [Year] |
| 6. Which etiology is considered the most probable cause of the hearing loss: | 6. Which etiology is considered the most probable cause of the hearing loss: |
| [unknown ▾] | [unknown ▾] unknown / genetic connexon / genetic: Usher syndrome / genetic other than above / neonatal infection: CMV / neonatal infection other than CMV / meningitis / ototoxicity / Cochlear malformation: Large Vestibular Aqueduct / Cochlear malformation: other than LVA / trauma |

7. Audiometry (dB):

| | 250 Hz | 500 Hz | 1000 Hz | 2000 Hz | 4000 Hz |
|---|---|---|---|---|---|
| Last Unaided | dB | dB | dB | dB | dB |
| Last Aided | dB | dB | dB | dB | dB |

7. Audiometry

| | | | | | |
|---|---|---|---|---|---|
| Last Unaided | dB | dB | dB | dB | dB |
| Last Aided | dB | dB | dB | dB | dB |

| Right Ear | Left Ear |
|---|---|
| 8. Speech audiometry: maxiamal word discrimination score | 8. Speech audiometry: maxiamal word discrimination score |
| [ ] % | [ ] % |
| 9. Speech audiometry in noise (0 dB S/N): maximal word discrimination score | 9. Speech audiometry in noise (0 dB S/N): maximal word discrimination score |
| [ ] % | [ ] % |

Copy information of right ear to left ear.

## FIGURE 2

Give Input

FIGURE 3

# FIGURE 4

| Your Input | |
|---|---|
| Patient | MISA00001 |
| Speech Algorithm | ACE |
| tNRT E10: | 190 |
| tNRT E15: | 190 |
| tNRT E20 | 190 |
| Subjective T on E15 | 180 |
| Date: | 23 November 2001 |
| Formula Version | 6 2 |

The MAP

| | E22 | E21 | E20 | E19 | E18 | E17 | E16 | E15 | E14 | E13 | E12 | E11 | E10 | E9 | E8 | E7 | E6 | E5 | E4 | E3 | E2 | E1 |
|---|---|---|---|---|---|---|---|---|---|---|---|---|---|---|---|---|---|---|---|---|---|---|
| C-3 | 126 | 130 | 127 | 129 | 133 | 132 | 138 | 137 | 136 | 144 | 141 | 146 | 150 | 152 | 147 | 157 | 157 | 161 | 164 | 162 | 167 | 169 |
| T-3 | 107 | 103 | 109 | 106 | 103 | 107 | 108 | 106 | 104 | 104 | 110 | 107 | 106 | 105 | 107 | 107 | 113 | 111 | 118 | 121 | 120 | 123 |
| C-2 | 135 | 131 | 136 | 139 | 138 | 145 | 149 | 150 | 152 | 151 | 153 | 152 | 158 | 159 | 162 | 157 | 168 | 171 | 173 | 173 | 169 | 173 |
| T-2 | 119 | 120 | 118 | 113 | 118 | 117 | 120 | 116 | 120 | 114 | 115 | 110 | 115 | 119 | 112 | 121 | 118 | 118 | 127 | 128 | 125 | 133 |
| C-1 | 140 | 144 | 144 | 145 | 143 | 149 | 151 | 157 | 160 | 158 | 158 | 162 | 160 | 169 | 168 | 168 | 170 | 174 | 171 | 179 | 177 | 179 |
| T-1 | 124 | 120 | 119 | 123 | 126 | 127 | 124 | 120 | 121 | 121 | 120 | 127 | 127 | 125 | 121 | 130 | 130 | 127 | 128 | 131 | 133 | 133 |
| C1 | 145 | 150 | 149 | 152 | 152 | 159 | 156 | 164 | 162 | 168 | 166 | 173 | 172 | 172 | 179 | 181 | 178 | 178 | 183 | 186 | 185 | 186 |
| T1 | 129 | 130 | 134 | 126 | 135 | 131 | 130 | 133 | 133 | 127 | 128 | 134 | 132 | 133 | 132 | 130 | 138 | 138 | 141 | 141 | 141 | 147 |
| C2 | 151 | 154 | 158 | 156 | 160 | 166 | 168 | 164 | 171 | 168 | 172 | 173 | 177 | 176 | 178 | 185 | 184 | 184 | 185 | 190 | 191 | 193 |
| T2 | 136 | 139 | 134 | 135 | 131 | 133 | 133 | 135 | 133 | 130 | 135 | 131 | 139 | 139 | 134 | 136 | 141 | 143 | 148 | 148 | | |
| C3 | 159 | 161 | 163 | 166 | 162 | 166 | 167 | 175 | 176 | 172 | 180 | 177 | 182 | 180 | 190 | 192 | 190 | 192 | 190 | 200 | 203 | 204 |
| T3 | 132 | 140 | 138 | 140 | 141 | 137 | 140 | 140 | 134 | 138 | 132 | 137 | 139 | 139 | 140 | 137 | 141 | 138 | 144 | 145 | 148 | 153 |
| C4 | 161 | 162 | 169 | 167 | 174 | 177 | 176 | 179 | 181 | 185 | 182 | 184 | 188 | 187 | 193 | 192 | 191 | 199 | 196 | 201 | 205 | 207 |
| T4 | 142 | 135 | 144 | 145 | 145 | 143 | 137 | 139 | 140 | 136 | 142 | 139 | 136 | 141 | 143 | 142 | 141 | 143 | 151 | 150 | 151 | 151 |
| C5 | 163 | 173 | 171 | 171 | 180 | 181 | 178 | 186 | 187 | 183 | 188 | 191 | 190 | 197 | 201 | 202 | 204 | 203 | 205 | 204 | 210 | 211 |
| T5 | 143 | 141 | 141 | 143 | 138 | 144 | 140 | 140 | 147 | 148 | 146 | 141 | 146 | 146 | 150 | 147 | 152 | 148 | 151 | 148 | 155 | 160 |
| C6 | 169 | 179 | 180 | 177 | 178 | 181 | 183 | 183 | 189 | 193 | 193 | 194 | 192 | 198 | 200 | 206 | 209 | 207 | 207 | 211 | 209 | 218 |
| T6 | 149 | 149 | 144 | 142 | 145 | 146 | 143 | 142 | 147 | 143 | 142 | 146 | 149 | 151 | 149 | 146 | 148 | 150 | 151 | 160 | 162 | 161 |
| C7 | 177 | 176 | 185 | 185 | 188 | 188 | 188 | 194 | 191 | 196 | 195 | 198 | 198 | 205 | 202 | 210 | 208 | 214 | 212 | 219 | 218 | 218 |
| T7 | 146 | 151 | 148 | 146 | 145 | 148 | 147 | 147 | 148 | 152 | 144 | 153 | 150 | 150 | 154 | 155 | 151 | 154 | 162 | 162 | 164 | |
| C8 | 186 | 187 | 183 | 185 | 190 | 192 | 197 | 197 | 197 | 200 | 204 | 202 | 206 | 212 | 214 | 210 | 217 | 214 | 218 | 219 | 223 | 222 |
| T8 | 153 | 153 | 149 | 149 | 145 | 153 | 154 | 153 | 151 | 154 | 149 | 150 | 146 | 149 | 150 | 153 | 152 | 151 | 158 | 164 | 162 | 159 |
| C9 | 189 | 187 | 192 | 189 | 195 | 196 | 199 | 204 | 205 | 207 | 209 | 212 | 208 | 212 | 213 | 221 | 222 | 218 | 226 | 223 | 228 | 226 |
| T9 | 151 | 148 | 145 | 151 | 146 | 149 | 151 | 151 | 144 | 152 | 150 | 152 | 153 | 150 | 154 | 155 | 150 | 156 | 152 | 159 | 163 | 164 |

**European Patent Office**

## DECLARATION

which under Rule 45 of the European Patent Convention
shall be considered, for the purposes of subsequent
proceedings, as the European search report

**Application Number**

EP 02 44 7028

| The Search Division considers that the present application, does not comply with the provisions of the EPC to such an extent that it is not possible to carry out a meaningful search into the state of the art on the basis of all claims<br><br>Reason: | CLASSIFICATION OF THE APPLICATION (Int.Cl.7)<br><br>A61N1/36 |
|---|---|

A meaningful search is not possible on the basis of all claims because all claims are directed to: diagnostic and therapeutical methods practised on the human or animal body - Article 52 (4) EPC (claims 1-14); program for computers - Article 52 (2)(c) EPC (claims 15-17); device with no technical features (claim 18).

The applicant's attention is drawn to the fact that a search may be carried out during examination following a declaration of no search under Rule 45 EPC, should the problems which led to the declaration being issued be overcome (see EPC Guideline C-VI, 8.5).
---
-----

| Place of search | Date | Examiner |
|---|---|---|
| THE HAGUE | 8 July 2002 | Ferrigno, A |

EPO FORM 1504 (P04C37)